# EUROPEAN PATENT APPLICATION

(11) **EP 4 691 461 A1**
(43) Date of publication of application: **11.02.2026**
(21) Application number: 25194937.6
(22) Date of filing: 08.08.2025
(51) Int. Cl.: A61K 31/192, A61P 31/14, A61P 25/28

(54) **BENZOATE FOR USE IN PREVENTING OR TREATING COGNITIVE DYSFUNCTION CAUSED BY LONG COVID**

(30) Priority: 09.08.2024 US 202463681271 P
(71) Applicant: Lane, Hsien-Yuan, Taichung City 406040 (TW); Lin, Chieh-Hsin, Kaohsiung City 833 (TW); Excelsior Pharmatech Labs, Taipei City 115011 (TW)
(72) Inventor: LANE, Hsien-Yuan, 406040 Taichung City (TW); LIN, Chieh-Hsin, 833401 Kaohsiung City (TW)
(74) Representative: Icosa

(57) **Abstract**

The present disclosure provides a method of preventing or treating cognitive dysfunction in a subject in need thereof, including administering to the subject an effective amount of a benzoic acid salt.

## Description

### BACKGROUND

### 1. Technical Field

The present disclosure relates to preventing or treatment of cognitive dysfunction, and particularly to methods for preventing or treating cognitive dysfunction caused by long COVID with benzoates.

### 2. Description of Associated Art

COVID-19 infection has claimed millions of lives globally; furthermore, it may remain a chronic condition for the survivors (Richardson et al. 2020). Concern over COVID-19's long-term consequences on many human attributes, especially neurocognitive functions, is increasing (Helms et al. 2020; Yang et al. 2021).

Many individuals infected with COVID-19 had mild symptoms or appeared asymptomatic. However, a number of patients suffer from a wide range of symptoms, including cognitive dysfunction, which are much longer than usual viral infections (Crivelli et al. 2022). WHO defines "Post-COVID-19 condition" as symptoms that appear three months following infection with SARS- CoV-2 and last for at least two months, with no other diagnosis being able to account for them (Cabrera Martimbianco et al. 2021). Instead, the term "Long COVID" is a more commonly used. Three main symptom clusters with long-term COVID-19 include lethargy, cognitive problems, and dyspnea (Slomski 2022).

Among the long COVID syndrome, "brain fog" is a very common neuropsychiatric syndrome. It describes a wide range of cognitive symptoms including slow or sluggish thinking, poor concentration, fuzzy thoughts, memory deficits or forgetfulness, feeling confused, lost words, mental fatigue and others (Heesakkers et al. 2022). In general, the individuals with COVID-19 infection show lower cognitive function than healthy individuals (Crivelli et al. 2022).

Approximately 10% people affiliated with COVID-19 had long COVID (Ladds et al. 2020). Cognitive problem occurred in 35.4% of long COVID cases (Douaud et al. 2022). Individuals who were hospitalized to the hospital due to an acute infection need a longer time to recover (about 15.1% patients continued to experience long COVID after one year of infection) (Wulf Hanson et al. 2022).

The brain fog can seriously impact the life quality of those patients, the medical system, and the society (Murtas et al. 2021; Heesakkers et al. 2022). The patients usually experience decreased ability to perform daily tasks, and many remain unable to work (Callan et al. 2022; Wulf Hanson et al. 2022), thereby increasing their medical needs and compromising associated clinical capacity and occupational productivity (Ladds et al. 2020; Callan et al. 2022).

Some general practices have been suggested to help patients recover from brain fog, such as exercise, good sleep, healthy diet, brain exercise (for example, playing games) and etc. However, the efficacy of these general practices was uncertain. A considerable portion of patients still suffer from brain fog (Krishnan et al. 2022).

Some researchers treated long COVID using noninvasive brain stimulation (NIBS) techniques based on the hypothesis of improved blood vessel regulation (Sabel et al. 2021). Although several researchers found that some patients had improvement in the cognitive function after NIBS treatment, the results have been inconclusive due to the open-labelled trials with modest sample sizes. Moreover, some patients' cognitive symptoms relapsed after discontinuation of NIBS.

Therefore, there is a pressing need to create safer and more effective treatments for long COVID-19 related cognitive impairment (Hampshire et al. 2022).

### SUMMARY

In view of the foregoing, the present disclosure provides a method of preventing or treating cognitive dysfunction in a subject in need thereof, comprising administering to the subject an effective amount of a benzoic acid salt.

In another aspect, the present disclosure further provides a use of benzoic acid salt in the manufacture of a composition for preventing or treating cognitive dysfunction.

In another aspect, the present disclosure further provides a composition for use in preventing or treating cognitive dysfunction in a subject in need thereof, wherein the composition comprises a benzoic acid salt and a pharmaceutically acceptable excipient thereof.

In one embodiment of the present disclosure, the benzoic acid salt is sodium benzoate, potassium benzoate, calcium benzoate, 2-aminobenzoate, 3-aminobenzoate, 4-aminobenzoate, benzyl benzoate, or methyl benzoate.

In another embodiment of the present disclosure, the benzoic acid salt is sodium benzoate.

In one embodiment of the present disclosure, the cognitive dysfunction is caused by long COVID.

In one embodiment of the present disclosure, the cognitive dysfunction is caused by microglia activation, systemic inflammation, or neuroinflammatory induced by COVID.

In one embodiment of the present disclosure, the benzoic acid salt may be administered to the subject in an amount ranging from 200 mg/day to 3000 mg/day, such as from 250 mg/day to 1500 mg/day, from 450 mg/day to 1000 mg/day, and from 500 mg/day to 750 mg/day.

In one embodiment of the present disclosure, the benzoic acid salt is administered one to four times per day.

In one embodiment of the present disclosure, the benzoic acid salt may be administered to the subject in a period ranging from 1 month to 1 years, such as from 8 weeks to 6 months. In another embodiment of the present disclosure, the benzoic acid salt is administered to the subject in a period of around 6 months.

Accordingly, it is provided a favorable pharmacological treatment for long COVID-related cognitive dysfunction.

### DETAILED DESCRIPTION OF THE EMBODIMENTS

The following examples are used to exemplify the present disclosure. A person of ordinary skills in the art can understand the other advantages of the present disclosure, based on the disclosure of the present specification. The present disclosure can also be implemented or applied as described in different specific examples.

While the respiratory system is the primary target of COVID, the infected patients can also suffer neurocognitive symptoms (Theoharides et al. 2021). Not only moderate-severe but also mild COVID-19 may alter the brain (Abbasi 2022). There are several possible ways to cause brain fog from long COVID (Yang et al. 2021); for example, COVID infection can alter choroid plexus cell types and brain structures, such as gray matter over parahippocampal gyrus, the orbitofrontal cortex, the primary olfactory cortex, and decrease whole-brain volume (Yang et al. 2021; Abbasi 2022; Douaud et al. 2022; Huang et al. 2022).

Regarding the molecular mechanism, COVID induces microglia activation, systemic inflammation, and neuroinflammatory changes, thereby affecting brain and cognitive function (Steardo et al. 2020; Boldrini et al. 2021; Theoharides et al. 2021).

Moreover, COVID also induces oxidative stress, thereby causing neurotoxicity and hampering neurocognitive function (Chernyak et al. 2020; Laforge etl al. 2020).

Excessively activated microglia generate numerous proinflammatory cytokines, which eventually damage neurons and cause neurodegeneration (Kaur et al. 2019). Sodium benzoate decreased inflammatory response in rodents (Brahmachari and Pahan, 2007; Brahmachari et al. 2009). Furthermore, by preventing glia activation in animals, sodium benzoate was able to maintain cognitive performance (Modi et al. 2015).

The current project will explore the following theme: assessing whether sodium benzoate therapy can help people with long COVID improve their cognitive performance.

It is further noted that, as used in this specification, the singular forms "a," "an," and "the" include plural referents unless expressly and unequivocally limited to one referent. The term "or" is used interchangeably with the term "and/or" unless the context clearly indicates otherwise.

The present disclosure provides methods of preventing or treating cognitive dysfunction in a subject in need thereof, comprising administering to the subject an effective amount of a benzoic acid salt.

As used herein, the term "treating" or "treatment" refers to administration of an effective amount of a benzoic acid salt to a subject in need thereof with the purpose of cure, alleviate, relieve, remedy, ameliorate, or prevent the disease, the symptoms thereof, or the predisposition towards it. Such a subject can be identified by a health care professional based on results from any suitable diagnostic method.

In one embodiment of the present disclosure, the subject to be treated by the method of the present disclosure suffers from cognitive dysfunction. In a further embodiment of the present disclosure, the cognitive dysfunction is caused by long COVID.

Benzoic acid occurs naturally in many plants and animals. It is therefore a natural constituent of many foods, including milk products (IPCS 1993). Benzoic acid and sodium benzoate are also legal food additives in USA (Joint FAO/WHO Expert Committee on Food Additives. 1965, 1973), Taiwan (Department of Health), and World Health Organization (IPCS 1993), and are widely used in manufacturing fruit jelly, butter, soy-bean sauce, processed meat, etc.

As used herein, the term "effective amount" refers to a therapy amount that is sufficient to result in prevention of the development, recurrence, or onset of cognitive dysfunction caused by long COVID and one or more symptoms thereof, to enhance or improve the prophylactic effect(s) of another therapy, reduce the severity, the duration of the disorder, ameliorate one or more symptoms of the disorder, prevent the advancement of cognitive dysfunction is caused by long COVID, and/or enhance or improve the therapeutic effect(s) of another therapy.

In some embodiments of the present disclosure, the effective amount of the benzoic acid salt may range from 200 mg/day to 3000 mg/day. In an embodiment, a lower limit of the dosage may be 200 mg/day, 225 mg/day, 250 mg/day, 275 mg/day, 300 mg/day, 325 mg/day, 350 mg/day, 400 mg/day, 450 mg/day, 500 mg/day, 550 mg/day, 600 mg/day, 700 mg/day, 750 mg/day, or 800 mg/day and an upper limit of the dosage may be 3000 mg/day, 2800 mg/day, 2500 mg/day, 2200 mg/day, 2000 mg/day, 1800 mg/day, 1500 mg/day, 1200 mg/day, 1000 mg/day, 900 mg/day, 800 mg/day, 750 mg/day, 700 mg/day, or 600 mg/day. For example, the dosage of the benzoic acid salt may be 200 mg/day to 3000 mg/day, 250 mg/day to 1500 mg/day, 450 mg/day to 1000 mg/day, 500 mg/day to 750 mg/day, 550 mg/day to 1000 mg/day, 600 mg/day to 800 mg/day, about 1000 mg/day, about 900 mg/day, about 750 mg/day, or about 500 mg/day.

As used herein, when a number or a range is recited, a person having ordinary skill in the art understands that it intends to encompass an appropriate, reasonable range for the particular field related to the disclosure.

By reciting at least 200 mg to 3000 mg, it meant that all integer unit amounts within the range are specifically disclosed as part of the disclosure. Thus, 200, 201, 202 ... 250, 251, 252 ... 1000, 1001, 1002 ... 2997, 2998, 2999 and 3000 unit amounts are included as embodiments of the present disclosure.

In some embodiments of the present disclosure, the administration of the benzoic acid salt may be conducted one to four times per day, for example, once per day, twice per day, 3 times per day, or 4 times per day. In an embodiment, the administration of the benzoic acid salt may be conducted once per day.

In some embodiments of the present disclosure, the benzoic acid salt may be administered to the subject in a period sufficient to prevent or treat cognitive dysfunction caused by long COVID. The sufficient period may depend on the species, gender, body weight or age of the subject, the stage, symptom or severity of the disease, and the routes, timing or frequency of the administration. In some embodiments of the present disclosure, the administration of the benzoic acid salt is daily over at least 1 month. For example, the period of administration of the benzoic acid salt may last for 1, 2, 3, 4, or 6 months, or 1, 2, 3 or 4 years, or even longer, as long as no side effect occurs during the treatment period. In the exemplary embodiments of the present disclosure, the period may be in a range of from 1 month to 1 years. In another embodiment, the period ranges from 8 weeks to 6 months. In yet another embodiment, the administration of the benzoic acid salt is daily for 6 months.

In certain embodiments of the present disclosure, the method involves the use of benzoic acid, benzoic acid salt, or a derivative thereof, which can be selected from the group consisting of benzoic acid, sodium benzoate, potassium benzoate, calcium benzoate, 2-aminobenzoate, 3-aminobenzoate, 4-aminobenzoate, benzyl benzoate, and methyl benzoate.

In certain embodiments of the present disclosure, the benzoic acid salt is administered in an oral dosage form. In some embodiments of the present disclosure, the benzoic acid salt administered to the subject may be contained in a pharmaceutical composition. The pharmaceutical composition of the present disclosure comprises a benzoic acid salt and a pharmaceutically acceptable excipient thereof. In an embodiment, the composition of the present disclosure is formulated in a form suitable for oral administration, and thus the composition may be administered to the subject by oral delivery. Alternatively, the composition may be formulated in a form of dry powder, a tablet, a lozenge, a capsule, granule, or a pill. The pharmaceutically acceptable excipient includes, but is not limited to, a filler, a binder, a preservative, a disintegrating agent, a lubricant, a suspending agent, a wetting agent, a solvent, a surfactant, an acid, a flavoring agent, polyethylene glycol (PEG), alkylene glycol, sebacic acid, dimethyl sulfoxide, an alcohol, or any combination thereof.

The pharmaceutical composition of the present disclosure may only comprise the benzoic acid salt as an active ingredient for preventing or treating cognitive dysfunction caused by long COVID. In other words, the benzoic acid salt serves as the only active ingredient for preventing or treating cognitive dysfunction caused by long COVID in the composition. In this embodiment, the present disclosure provides a safe and effective therapy for preventing or treating cognitive dysfunction caused by long COVID by the use of the benzoic acid salt alone as the active ingredient.

Alternatively, in another embodiment, the composition may be administered to a subject in combination with another active ingredient unless the effect of the disclosure is inhibited. The benzoic acid salt and another active ingredient may be provided in a single composition or in separate compositions.

Many examples have been used to illustrate the present disclosure. The examples below should not be taken as any limit to the scope of the present disclosure.

### EXAMPLE

The present disclosure examined the efficacy and safety of benzoic acid salt for the treatment of cognitive dysfunction of the persons with long COVID.

The inventors conducted a 24-week open-label sodium benzoate clinical trial. 11 individuals long COVID-induced cognitive deficits have been recruited and treated with 250-3000 mg/day of sodium benzoate for 24 weeks. Alzheimer's disease assessment scale-cognitive subscale (ADAS-cog, the primary outcome) was measured every eight weeks. Additional cognition composite was measured at baseline and endpoint. Among the 11 patients, 2 patients ended at the final dose of 500 mg/day of sodium benzoate, 6 at 750 mg/day, and 3 at 1000 mg/day. The sodium benzoate dosage plan is determined by the amounts found in the previously described study (Lin, Chen et al. 2014, Lane, Tu et al. 2021), where sodium benzoate appeared safe and efficacious after 24-week therapy among the individuals with early-phase Alzheimer dementia.

### Assessments

The primary outcome was the Alzheimer's disease assessment scale-cognitive subscale (ADAS-cog) (43) measured at weeks 0, 8, 16, and 24. ADAS-cog is the most popular cognitive assessment instrument used in AD clinical trials. It consists of 11 tasks, including word recall, naming, commands, constructional praxis, ideational praxis, orientation, word recognition, instructions remembering, spoken language ability, word-finding difficulty and comprehension. Its scores range from 0 (best) to 70 (worst).

**Table 1. The change of ADAS-cog score over 24 weeks in 11 patients with long COVID-19 cognitive deficits using paired t-test.**

| ADAS-cog | Mean ± SD (N=11) |
|---|---|
| Baseline | 12.8 ± 11.0 (N=11) |
| Endpoint | 8.5 ± 8.6 (N=11) |
| P value | 0.026 |

| | |
|---|---|
| Abbreviation: ADAS-cog, Alzheimer's disease assessment scale-cognitive subscale | |

As shown in Table 1, in this pilot, open-label clinical trial, their cognitive function improved after 24-week treatment of sodium benzoate, falling from the mean score of 12.8 ± 11.0 (SD) in ADAS-cog at baseline to 8.5 ± 8.6 at endpoint (p=0.026). Moreover, the safety profiles were excellent, and no patient was dropped out due to side effect. This encouraging finding is the first time to show the efficacy of sodium benzoate in the treatment of brain fog of long COVID syndrome.

The foregoing descriptions of the detailed embodiments are only illustrated to disclose the principle and functions of the present disclosure and do not intend to restrict the scope of the present disclosure. It should be understood to those skilled in the art that all modifications and variations according to the spirit and principle of the present disclosure should fall within the scope of the appended claims. It is intended that the specification and examples are considered as exemplary only, with a full scope of the disclosure being indicated by the following claims.

The references listed below in the application are each incorporated by reference as if they were incorporated individually.
D Abbasi, J. (2022). JAMA 327(14): 1321-1322.
Batshaw, M. L. and S. W. Brusilow (1981). J Inherit Metab Dis 4(4): 231. Batshaw, M. L. and
P. S. Monahan (1987). Enzyme 38(1-4): 242-250.
Boldrini, M., P. D. Canoll and R. S. Klein (2021). JAMA Psychiatry 78(6): 682-683.
Brahmachari, S., A. Jana and K. Pahan (2009). J Immunol 183(9): 5917-5927.
Brahmachari, S. and K. Pahan (2007). J Immunol 179(1): 275-283.
Cabrera Martimbianco, A. L., R. L. Pacheco, et al (2021). Int J Clin Pract 75(10): e14357.
Callan, C., E. Ladds, L. Husain, et al (2022). BMJ Open 12(2): e056366.
Chang, C. H., C. H. Lin, C. Y. Liu, C. S. Huang, S. J. Chen, W. C. Lin, H. T. Yang and H. Y. Lane (2021). J Psychopharmacol 35(3): 265-272.
Chernyak, B. V., E. N. Popova, A. S. Prikhodko, et al (2020). Biochemistry 85(12): 1543-1553.
Cohen, S., T. Kamarck and R. Mermelstein (1983). J Health Soc Behav 24(4): 385-396.
Crivelli, L., K. Palmer, I. Calandri, et al (2022). Alzheimers Dement 18(5): 1047-1066.
Cummings, J. L., D. P. Goldman, N. R. Simmons-Stern and E. Ponton (2022). Alzheimers Dement 18(3): 469-477.
Douaud, G., S. Lee, F. Alfaro-Almagro, et al (2022). Nature 604(7907): 697-707.
Faul, F., E. Erdfelder, A. Buchner and A. G. Lang (2009). Behav Res Methods 41(4): 1149-1160. Feillet, F. and J. V. Leonard (1998). J Inherit Metab Dis 21 Suppl 1: 101-111.
Green, T. P., R. P. Marchessault and D. K. Freese (1983). J Pediatr 102(5): 785-790. Hamad,
R. and S. Galea (2022). JAMA 328(1):17-18.
Hamilton, M. (1959). Br J Med Psychol 32(1): 50-55.
Hamilton, M. (1960). J Neurol Neurosurg Psychiatry 23: 56-62.
Hampshire, A., D. A. Chatfield, M. P. AM, A. Jolly, et al (2022). EClinicalMedicine 47: 101417.
Heesakkers, H., J. G. van der Hoeven, S. Corsten, et al (2022). JAMA 327(6): 559-565.
Helms, J., S. Kremer, H. Merdji, et al (2020). N Engl J Med 382(23): 2268-2270.
Huang, C. C., I. H. Wei, C. L. Huang, K. T. Chen, M. H. Tsai, P. Tsai, R. Tun, K. H. Huang, Y. C. Chang, H. Y.
Huang, Y., Q. Ling, A. Manyande, D. Wu and B. Xiang (2022). Front Neurosci 16: 855868.
Kaur, D., V. Sharma and R. Deshmukh (2019). Inflammopharmacology 27(4): 663-677.
Kessler, R. C., C. J. Ruhm, V. Puac-Polanco, et al (2022). JAMA Netw Open 5(6): e2217223.
Krishnan, K., Y. Lin, K. M. Prewitt, et al (2022). J Health Serv Psychol 48(1): 31-38.
Kubota, K. and T. Ishizaki (1991). Eur J Clin Pharmacol 41(4): 363-368.
Ladds, E., A. Rushforth, S. Wieringa, et al (2020). BMC Health Serv Res 20(1): 1144.
Laforge, M., C. Elbim, C. Frere, et al (2020). Nat Rev Immunol 20(9): 515-516.
Lane, H. Y. and W. H. Chang (1998). J Clin Psychiatry 59(8): 430-431.
Lane, H. Y., Y. C. Chang, Y. C. Liu, C. C. Chiu and G. E. Tsai (2005). Arch Gen Psychiatry 62(11): 1196-1204.
Lane, H. Y., C. L. Huang, P. L. Wu, et al (2006). Biol Psychiatry 60(6): 645-649. Lane, H. Y., & Lin, C. H. (2023). Int J Neuropsychopharmacol 26(1), 1-8.
Lane, H. Y., C. H. Lin, M. F. Green, et al (2013). JAMA Psychiatry 70(12): 1267-1275.
Lane, H. Y., C. H. Lin, Y. J. Huang, et al (2010). Int J Neuropsychopharmacol 13(4): 451-460.
Lane, H. Y., Y. C. Liu, C. L. Huang, et al (2008). Biol Psychiatry 63(1): 9-12.
Lane, H. Y., C. H. Tu, W. C. Lin and C. H. Lin (2021). Int J Neuropsychopharmacol 24(5): 392-399.
Lane, H. Y., S. H.Wang and Lin, C. H. (2023 Aug). Psychiatry Res 326: 115288. Lane, H. Y., S. H.Wang and Lin, C. H. (2023 Oct). Psychiatry Res 328: 115461.
Lin, C. H., H. T. Chang, Y. J. Chen, C. H. Lin, C. H. Huang, R. Tun, G. E. Tsai and H. Y.
Lane (2014). Mol Psychiatry 19(6): 636-637.
Lin, C. H., P. K. Chen, Y. C. Chang, L. J. Chuo, Y. S. Chen, G. E. Tsai and H. Y. Lane (2014).
Biol Psychiatry 75(9): 678-685.
Lin, C. H., C. C. Chiu, C. H. Huang, H. T. Yang and H. Y. Lane (2019). Sci Rep 9(1): 13221.
Lin, C. H. and H. Y. Lane (2019). Front Pharmacol 10: 540. Lin, C. H. and H. Y. Lane (2021).
Antioxidants 10(11):1839.
Lin, C. H. and H. Y. Lane (2022). Int J Neuropsychopharmacol 16;25(8):660-665.
Lin, C. H., H. Y. Lane and G. E. Tsai (2012). Pharmacol Biochem Behav 100(4): 665-677.
Lin, C. H., C. H. Lin, Y. C. Chang, Y. J. Huang, P. W. Chen, H. T. Yang and H. Y. Lane (2018).
Biol Psychiatry 84(6): 422-432.
Lin, C. H., P. P. Lin, C. Y. Lin, C. H. Lin, C. H. Huang, Y. J. Huang and H. Y. Lane (2016). J Psychiatr Res 72: 58-63.
Lin, C. H., H. T. Yang, C. C. Chiu and H. Y. Lane (2017). Sci Rep 7(1): 14849.
Lin, C. H., H. T. Yang and H. Y. Lane (2019). Pharmacol Biochem Behav 185: 172760.
Lingjaerde, O., U. G. Ahlfors, P. Bech, et al (1987). Acta Psychiatr Scand Suppl 334: 1-100.
Modi, K. K., A. Roy, S. Brahmachari, et al (2015). PLoS One 10(6): e0130398.
Mohs, R. C. and L. Cohen (1988). Psychopharmacol Bull 24(4): 627-628.
Murtas, R., N. Morici, C. Cogliati, et al (2021). JMIR Public Health Surveill 7(11): e29504.
O'Connor, J. E., M. Costell and S. Grisolia (1987). Biochem Biophys Res Commun 145(2): 817- 824.
Perneczky, R., C. Pohl, C. Sorg, et al (2006). Age Ageing 35(3): 240-245.
Rahman, M. A., K. Islam, S. Rahman et al (2021). Mol Neurobiol 58(3): 1017-1023. Reitan, R. M. (1958). Perceptual and Motor Skills 8: 271-276.
Richardson, S., J. S. Hirsch, M. Narasimhan, et al (2020). JAMA 323(20): 2052-2059. Sabel, B. A., W. Zhou, F. Huber, et al (2021). Restor Neurol Neurosci 39(6): 393-408.
Schneider, L. S., J. T. Olin, R. S. Doody, et al (1997). Alzheimer Dis Assoc Disord 11 Suppl 2: S22- 32.
Silver, H., P. Feldman, W. Bilker, et al (2003). Am J Psychiatry 160(10): 1809-1816. Slomski, A. (2022). JAMA 327(1): 26.
Steardo, L., Jr., L. Steardo and A. Verkhratsky (2020). Transl Psychiatry 10(1): 261.
Sternberg, S. (1966). Science 153(736): 652-654.
Theoharides, T. C., C. Cholevas, K. Polyzoidis, et al (2021). Biofactors 47(2): 232-241.
Tremblay, G. C. and I. A. Qureshi (1993). Pharmacol Ther 60(1): 63-90.
Tsai, G., H. Y. Lane, P. Yang, et al (2004). Biol Psychiatry 55(5): 452-456. Tsuang, H. C., S.
H. Lin, S. K. Liu, et al (2006). Schizophr Res 87(1-3): 172-180.
Wang, H., R. Qin, J. Zhang and Y. Chen (2021). Clin Neurol Neurosurg 201: 106414.
Wechsler, D. (1997). Psychological Association, San Antonio, TX.
Wu, P. L., H. S. Tang, H. Y. Lane, C. A. Tsai and G. E. Tsai (2011). J Clin Psychopharmacol 31(3): 369-374.
Wulf Hanson, S., C. Abbafati, J. G. Aerts, et al. (2022). medRxiv:2022.05.26.22275532.
Yang, A. C., F. Kern, P. M. Losada, et al (2021). Nature 595(7868): 565-571. Zeger, S. L. and K. Y. Liang (1986). Biometrics 42(1): 121-130.

## Claims

1. A pharmaceutical composition for use in preventing or treating cognitive dysfunction in a subject in need thereof, wherein the pharmaceutical composition comprises an effective amount of a benzoic acid salt, and wherein the cognitive dysfunction is caused by long COVID.

2. The pharmaceutical composition for use according to claim 1, wherein the benzoic acid salt is sodium benzoate, potassium benzoate, calcium benzoate, 2-aminobenzoate, 3-aminobenzoate, 4-aminobenzoate, benzyl benzoate, or methyl benzoate.

3. The pharmaceutical composition for use according to claim 1, wherein the benzoic acid salt is sodium benzoate.

4. The pharmaceutical composition for use according to claim 1, wherein the cognitive dysfunction is caused by microglia activation, systemic inflammation, or neuroinflammatory induced by COVID.

5. The pharmaceutical composition for use according to claim 1, wherein the benzoic acid salt is administered to the subject in an amount of from 200 mg/day to 3000 mg/day.

6. The pharmaceutical composition for use according to claim 1, wherein the benzoic acid salt is administered to the subject in an amount of from 250 mg/day to 1500 mg/day.

7. The pharmaceutical composition for use according to claim 1, wherein the benzoic acid salt is administered to the subject in an amount ranging from 450 mg/day to 1000 mg/day.

8. The pharmaceutical composition for use according to claim 1, wherein the benzoic acid salt is administered to the subject in an amount ranging from 500 mg/day to 750 mg/day.

9. The pharmaceutical composition for use according to claim 1, wherein the benzoic acid salt is administered one to four times per day.

10. The pharmaceutical composition for use according to claim 1, which is administered to the subject in a period ranging from 1 month to 1 year.

11. The pharmaceutical composition for use according to claim 1, which is administered to the subject in a period ranging from 8 weeks to 6 months.
